# EUROPEAN PATENT SPECIFICATION

(11) **EP 4 109 103 B1**
(45) Date of publication and mention of the grant of the patent: **20.03.2024**
(21) Application number: 21180857.1
(22) Date of filing: 22.06.2021
(51) Int. Cl.: G01N 33/68, G01N 33/86

(54) **METHOD FOR DETECTION OF HEPARIN-INDUCED THROMBOCYTOPENIA ANTIBODIES**
VERFAHREN ZUR DETEKTION VON HEPARININDUZIERTEN THROMBOZYTOPENIE-ANTIKÖRPERN
PROCÉDÉ DE DÉTECTION DES ANTICORPS CONTRE LA THROMBOCYTOPÉNIE INDUITE PAR L'HÉPARINE

(43) Date of publication of application: 28.12.2022
(73) Proprietor: Institut für Bioprozess- und Analysenmesstechnik e.V., 37308 Heilbad Heiligenstadt (DE)
(72) Inventor: NGUYEN, Thi Huong, 37308 Heilbad Heiligenstadt (DE); CHEN, Li-Yu, 37308 Heilbad Heiligenstadt (DE); APTE, Gurunath, 37308 Heilbad Heiligenstadt (DE)
(74) Representative: Maikowski & Ninnemann Patentanwälte Partnerschaft mbB

(56) References cited:
- WO-A1-2019/202395
- WO-A2-2013/165969
- THI-HUONG NGUYEN ET AL: "Distinct Binding Characteristics of Pathogenic Anti-Platelet Factor-4/Polyanion Antibodies to Antigens Coated on Different Substrates: A Perspective on Clinical Application", ACS NANO, vol. 12, no. 12, 12 December 2018 (2018-12-12), pages 12030-12041, XP055625056, US ISSN: 1936-0851, DOI: 10.1021/acsnano.8b04487

## Description

### Field of the invention

The invention relates to a method for the detection of heparin-induced thrombocytopenia antibodies (HIT Abs) in a biological sample comprising the steps of
i) providing a solid phase support, wherein said solid support is coated with cells or cell membranes on the surface,
ii) binding of platelet factor 4 (PF4) to said cells on the solid phase support,
iii) incubating the solid phase support of step ii) with a biological sample,
iv) binding of HIT Abs which are present in said biological sample to PF4 on said cells on the solid phase support,
v) adhering a secondary anti-human antibody, which is preferably labeled; and which is capable of binding to the bound HIT Abs on said cells on the solid phase support, and
vi) detecting the presence of the secondary anti-human antibody,
wherein the detection of said secondary anti-human antibody is a positive indicator for the presence of HIT Abs in the biological sample, characterized in that said cells or cell membranes on the solid phase support are cancer cells or membranes from cancer cells. The invention further relates to the use of said method for diagnosing heparin-induced thrombocytopenia in a subject.

### Background of the invention

Heparin is the classic anticoagulant. However, the incidence of heparin-induced thrombocytopenia (HIT) ranges from 3 to 5% in unfractionated heparin (UFH) and 0.2-0.6% with low molecular weight heparin (LMWH) therapy.¹ HIT usually occurs within 4-15 days after heparin infusion but may occur as early as 1 day in cases with a prior history of heparin exposure.

HIT is caused by the development of some platelet-activating antibodies (HIT Abs) against platelet factor 4 (PF4) in complex with heparin (H). HIT Abs cross-link and activate platelets, monocytes, neutrophils, and bind to endothelial cells (EC) through binding with PF4/Heparin complexes or bound PF4/polyanions on cells, stimulating the clotting system. This leads to tissue factor expression that activates monocytes while activated platelets produce platelet-derived microparticles that accelerate thrombin generation, resulting in an increased risk for thrombosis or inducing procoagulant activity.^{2,3} This can induce the most frequent immune-mediated adverse drug reactions, the life-threatening autoimmune HIT.⁴ The disorder is associated with venous thromboembolic events HIT-thrombosis and a mortality rate up to 30%.⁵ It has been reported that 600,000 people per year develop HIT, which is double the number of breast cancer cases diagnosed annually in the US (US Cancer Statistics Working group), and nearly equal to the number of new cases of angina diagnosed each year.⁶

In severe Covid-19 patients, heparins including UFH and LMWH are recommended for the management of coagulopathy⁷ even at higher/double dose,⁸ as they appear to be associated with better prognosis.^{9,10} However, a 71.4% of the deaths in Covid-19 patients had abnormal coagulation profiles,^{15,16} while the incidence of thromboembolism in patients with severe coronavirus pneumonia is 25%.¹⁷ HIT Abs were detected in Covid-19 patients with heparin therapy.^{11, 34, 35} The incidence of HIT in Covid-19 patients at the intensive care unit is higher (1.16%) than for critically ill patients without Covid-19 (0.18%).^{12, 36} Recently, the individuals exposed to the ChAdOx1 nCoV-19 vaccine (AstraZeneca) also developed HIT antibodies, which in turn strongly activated platelets, causing thrombotic thrombocytopenia.^{37, 38, 39} HIT Abs activate platelets *via* FcγRIIa receptors which induced procoagulant platelets,¹³ As HIT Abs activate platelets and can trigger the clotting system, early detection of HIT antibodies to stop heparin therapy in patients is important to reduce thrombosis formation, especially in Covid-19 patients.

Failure to diagnose HIT can lead to catastrophic thrombosis if heparin therapy is continued. Therefore, a quick and accurate diagnosis of HIT must be made in case it occurs. The reliable test results can be tremendously helpful to a clinician who is called upon to make a management decision for both Covid-19 and non-Covid-19 patients.

The antibodies are initially formed after 5 or more days when a patient has been on heparin therapy. An immune response to a heparin dose may be observed within minutes or hours if the patient has had previous exposure to heparin and antibodies are already circulating. The diagnosis of HIT is difficult because its signs are non-specific. To date, early detection of HIT is still challenging, as available methods are either inaccurate in diagnosis, or required fresh blood platelets that are only available in some special laboratories or had high costs. During the Covid-19 pandemic, single test methods do not serve the urgent requirement as a huge number of patients need to be tested and re-tested frequently.

So far, HIT can be identified *via* the detection of HIT antibodies or *via* platelet activation. Two different assays can be used: i) antigen tests: the Enzyme-Linked Immunosorbent Assay (ELISA) and ii) functional assays: including the Serotonin-release assay (SRA)^{18,19} and the heparin-induced platelets aggregation assay (HIPA).²⁰ ELISA is a solid phase assay that uses PF4/Heparin complexes as targets for HIT antibodies. It is the most used test to detect human anti-PF4/Heparin antibodies (including both HIT and non-HIT Abs) which can be rapidly performed in clinical laboratories with high sensitivity.²¹ However, the ELISA is only suited to rule out HIT because it detects the mixture of both HIT and non-HIT antibodies. Around 50% of patients with positive results in this assay do not develop HIT.²² To confirm the patients with clinical HIT, the ELISA-positive samples must be retested in functional assays which require fresh blood platelets and technical skills while they are expensive and can be performed routinely in only a few specialized laboratories. Besides, several HIT test kits are also available in the market such as particle gel immune assays (PaGIA),^{23,24} STic,²⁵ AcuStar,²⁶ etc. However, a single kit package can be used for only several tests that turn out to be a very high cost for a large number of patients in the end. In comparison to ELISA, these methods show some limitations including i) high complexity, expensive, time-consuming, limited sensitivity, required specialized analyzer, fresh blood or donor platelets or platelet-rich plasma (PRP), not well suited to urgent tests or a large number of samples.

Overall, ELISA is the cheapest test, less complicated, relatively rapid with high sensitivity, and possible to perform large batches of samples at a single run with excellent negative prediction. It also allows quantitative detection of IgG-only (e.g., high level of OD may be associated with the likelihood of pathogenic HIT),²⁶ However, the specificity is low as many positive results will not define pathogenic HIT.²⁶ Meanwhile, the high cost and its limited availability worldwide make functional tests less practical from a laboratory perspective than other methods.

WO 2019/202395 discloses an ELISA method using platelets or platelet fractions for the detection of heparin-induced thrombocytopenia antibodies (HIT Abs). However, the method disclosed in WO 2019/202395 requires human blood platelet samples that is only available at some special Labs. Furthermore, platelets are one of the most sensitive cell types without nucleus that can be easily activated to release multiple proteins that may interfere with the detection of HIT antibodies. Besides, characteristics of platelets can differ widely depending on sample treatments, donors, and manipulation skills. This method is at the end not specific and simple enough to discriminate between HIT Abs and non-HIT Abs, and therefore, bears still the risk of false-positive or false-negative test results.

The objective of our invention is to overcome the obstacles of the methods in the prior state of the art and to provide an easy to handle and reliable method for detection of HIT Abs, wherein said method solves limitations in ELISA, functional tests, and other available assays.

### SUMMARY OF THE INVENTION

The objective of the invention is to overcome the obstacles of the methods in the prior state of the art and to provide an easy to handle and reliable method for detection of HIT Abs, wherein said method solves limitations in ELISA, functional tests, and other available assays.

The invention provides a method for the detection of heparin-induced thrombocytopenia antibodies (HIT Abs). The key of the invention is the finding that cancer cells or fragments thereof, such as membranes of cancer cells are highly negative-charged surfaces that allow binding of the positively charged platelet factor 4 (PF4) antigens *via* electrostatic interaction. HIT Abs bind strongly whereas non-HIT Abs adhere weakly to PF4/Heparin complexes. The interaction of non-HIT Abs is charge-related while HIT Abs have different binding characteristics.⁴⁰ For example, the monoclonal mimicking human HIT Abs, the KKO, intercalates in the open end of the PF4 tetramer whereas monoclonal mimicking human non-HIT Abs, the RTO, bind to PF4 monomers *via* electrostatic interaction. It has been shown that PF4 interferes with angiogenic growth factors bFGF-2 and VEGF165, activation of CXCR3B, interactions with integrins, interference with cell cycle, interactions with factors such as VEGF121 and CXCL8/IL-8, resulting in an inhibition of cell proliferation and migration. Thus, PF4 became a potential clinical anti-tumor agent.⁴¹ Our results showed no binding of RTO to PF4s bound on breast cancer cells, indicating that the positively charged PF4s were neutralized after binding to cell surfaces, resulting in no further available charges for the formation of electrostatic interaction between RTO and PF4. In contrast, this modification of surface charge did not affect the intercalation of KKO at the open end of PF4 tetramer. As HIT antibodies or non-HIT Abs have similar binding characteristics to PF4/Heparin complexes when comparing with KKO or RTO, respectively, they can be effectively detected using cancer cell-based membrane methods. The invention is based on the principle of the most widely used method in clinical laboratories, the enzyme-linked immunosorbent assay (ELISA) comprising i) culturing cells (in this case cancer cells) on ELISA plates or coating the surface of ELISA plates with cell membranes from cancer cells, ii) binding of PF4 to said cancer cells or cancer cell membranes, iii) binding of HIT Abs in diluted sera on cells, iv) adhering a labelled anti-human antibody that will bind to the bound HIT Abs on cells, and v) detecting the presence of the labelled anti-human antibody. Thereby, the HIT Abs bound to the secondary antibody will be identified. The invention provides a simple method with a relatively short protocol. The high specificity of detection and the cost-effectiveness confer the assay potential for commercialization for HIT diagnosis.

In a further embodiment, the invention relates to the use of the method for the detection of heparin-induced thrombocytopenia antibodies (HIT Abs) in a biological sample for diagnosing heparin-induced thrombocytopenia in a subject.

In a further embodiment, the invention provides a method for diagnosing heparin-induced thrombocytopenia in a subject comprising the detection of heparin-induced thrombocytopenia antibodies (HIT Abs) in a biological sample.

In a further embodiment, here provided but not part of the invention is a kit for the detection of heparin-induced thrombocytopenia antibodies (HIT Abs) in a biological sample.

### Brief description of the drawings

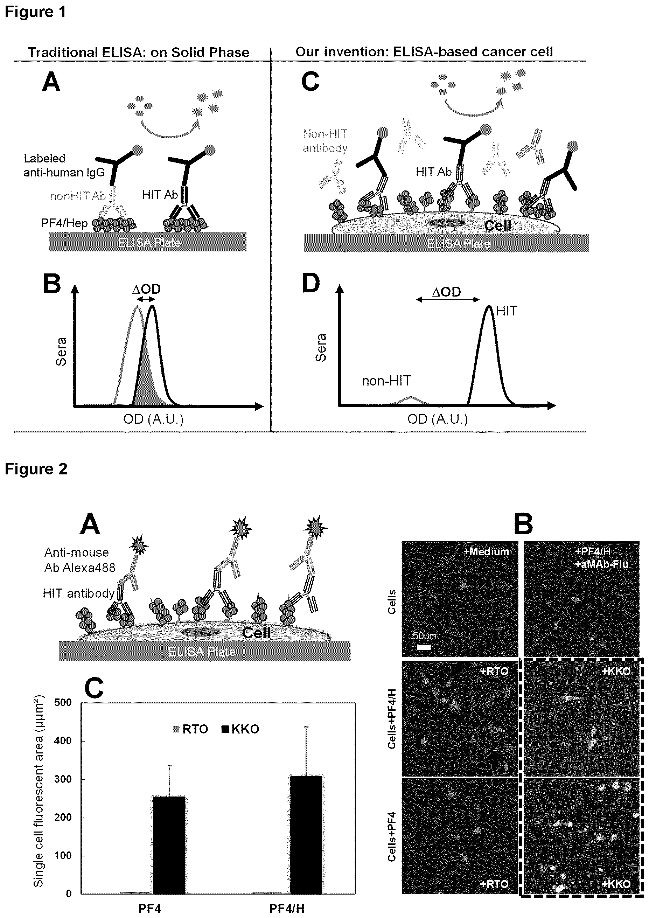

**Figure 1** shows a schematic illustration of a standard ELISA on a solid phase vs. the ELISA according to the invention using cancer cells or cancer cell membranes. (A) In the traditional ELISA method for detection of HIT antibodies, PF4/Heparin complexes are immobilized directly on a 96-well plate prior to adding human sera containing both HIT and non-HIT antibodies (Ab) for binding to PF4/Heparin complexes. Optical density (OD) of bound antibodies is detected using anti-human IgG peroxidase conjugate. (B) This method causes around 50% overlapped OD signal, indicating only around 50% accuracy for the detection of HIT antibodies. (C) Our invention is an ELISA-based cancer cell membrane. PF4s are immobilized on cancer cell membrane instead of 96-well plate while the following steps are similar to those in the traditional method (A). (D) Due to different binding characteristics, HIT antibodies could be detected from non-HIT antibodies.
**Figure 2** shows the visualization of the binding of HIT antibodies on cells. **(A)** PF4 coated cells on an ELISA plate were incubated with RTO or KKO, and anti-mouse-antibody-Alexa 488 (green) was added to detect bound antibodies on cells. **(B)** Cell nuclei were stained with DAPI (blue) and imaged by CLMS. Only KKO showed binding (bright) to both PF4 and PF4/Heparin coated cells. **(C)** Quantification of the signals showed a strong fluorescent area for KKO (dashed box) whereas RTO and other controls showed a minimal signal.

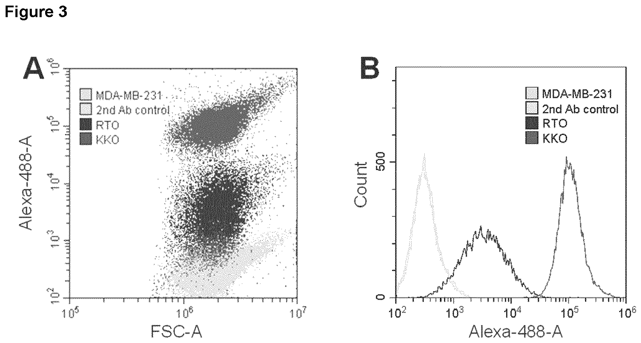

**Figure 3** shows the quantification of binding of HIT antibodies on cells by flow cytometry. **(A)** Histogram distributions show binding of both KKO (circles) and RTO (squares) on cells, but KKO bound much stronger than RTO which can be clearly distinguished while cells alone (open circles) and cells stained with the labeled secondary anti-mouse antibody Alexa-488 showed a much lower fluorescent signal. **(B)** Quantification of population density of fluorescent signal clearly shows the distinct distributions between KKO (solid) and RTO (dash) or controls (light gray, black).

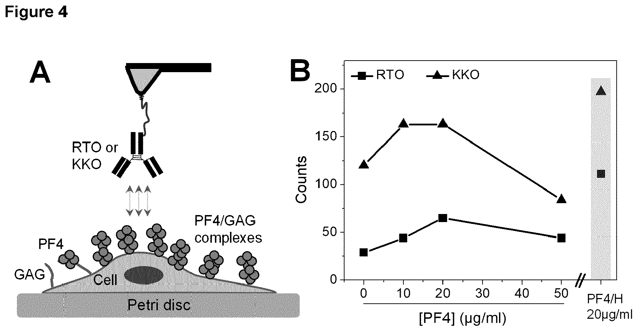

**Figure 4** **Label-free detection of HIT antibodies** by single-molecule force spectroscopy (SMFS). **(A)** Set up for measuring the interaction between RTO or KKO immobilized on the tip and PF4, PF4/H antigens coated on breast cancer cells. **(B)** At different PF4 concentrations or preformed PF4/H complexes, KKO shows a higher frequency of interaction (count number) than RTO.

### Detailed description of the invention

The problem of the invention is solved by a method for the detection of heparin-induced thrombocytopenia antibodies (HIT Abs) in a biological sample according to claim 1.

It has surprisingly been found by the inventors that cancer cells or fragments thereof, especially cell membranes of cancer cells, are highly effective in the binding of platelet factor 4 (PF4) antigens and thereby induce binding epitopes for capturing HIT Abs but not for non-HIT Abs, allowing an effective and specific detection of HIT Abs without detecting non-HIT Abs.

In particular, the method of the invention comprises the steps of:
i) providing a solid phase support, wherein said solid support is coated with cells or cell membranes on the surface,
ii) binding of platelet factor 4 (PF4) to said cells or cell membranes on the solid phase support,
iii) incubating the solid phase support of step ii) with a biological sample,
iv) binding of HIT Abs which are present in said biological sample to PF4 on said cells on the solid phase support,
v) adhering a secondary anti-human antibody, which is preferably labeled; and which is capable of binding to the bound HIT Abs on said cells or cell membranes on the solid phase support, and
vi) detecting the presence of the secondary anti-human antibody,
wherein the detection of said secondary anti-human antibody is a positive indicator for the presence of HIT Abs in the biological sample, characterized in that said cells or cell membranes on the solid phase support, are cancer cells or membranes from cancer cells.

When the solid support in step i) is coated with cancer cells, then, the method of the invention further comprises the step of
i) culturing cells on the solid phase support under conditions permitting the adherence of the cultured cells to the surface of the solid phase support.

In step ii) of the method of the invention, the binding of platelet factor 4 (PF4) to said cells or cell membranes on the solid phase support may be performed with or without fixation which is performed by incubating cells with a suitable agent and for a suitable time with 4% Paraformaldehyde (*PFA*) for 15 min at RT.

The method of the invention was tested with monoclonal HIT-like antibodies including RTO (does not induce HIT) and KKO (induces HIT). KKO is a monoclonal IgG2bκ antibody that binds specifically to human PF4/heparin complexes. RTO is a murine anti-human PF4 monoclonal antibody that does not require heparin for binding, reveals no obvious relationship in either the heavy- or the light chain immunoglobulin variable regions, both have molecular weights around 150 kDa. The Fc part of the KKO can bind to the FcyRlla on platelet membranes, activate, and induce platelet aggregation while the RTO does not have this platelet-activating function. Therefore, KKO recapitulates the antigenic and functional specificity of a subset of HIT antibodies and may, therefore, provide insight into the pathogenesis of thrombocytopenia and thrombosis in affected persons.³¹

In general, cells or cell membranes from any type of cancer cells can be used in the method of the invention likely due to binding ability of PF4 to various types of cell factors,^{32, 33} which differ from normal cells. The positively charged PF4 molecules, which form electrostatic interactions with the non-HIT Abs (e.g. the RTO)⁴², are neutralized after binding to cell surfaces, resulting in no further available charges for the formation of electrostatic interaction between RTO and PF4. In contrast, this change of PF4 do not affect the binding of HIT antibodies (e.g. the KKO). Suitable cancer cells or cancer cell membranes are, e.g., obtained from cancer cells selected from malignant (and preferably solid) tumors of epithelial or mesenchymal cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas and gliosarcomas such as glioblastoma multiforme.

Preferably, the cancer cells or cancer cell membranes are breast cancer cells or obtained from breast cancer cells. Most preferably, the cancer cells or cancer cell membranes are cells from the breast cancer cell line MDA MB 231 or are obtained from breast cancer cell line MDA MB 231.

The MDA-MB-231 cell line (isolated at M D Anderson from a pleural effusion of a patient with invasive ductal carcinoma) is commonly used to model late-stage breast cancer. This cell line is ER, PR, and E-cadherin negative and expresses mutated p53. In microarray profiling, the MDA-MB-231 cell genome clusters with the basal subtype of breast cancer. Since the cells also lack the growth factor receptor HER2, they represent a good model of triple-negative breast cancer. MDA-MB-231 cells are invasive in vitro and when implanted orthotopically produce xenografts that spontaneously metastasize to lymph nodes.

Cells of the cell line MDA-MB-231 can be obtained from well-known cell collections, such as the American Type Culture Collection (ATCC). Cell line MDA-MB-231 has accession no. ATCC HTB-26. A culture medium for growing cell line MDA-MB-231 and a protocol for handling the cell line are public information, which can be obtained from ATCC from the product sheet for ATCC HTB-26.

According to the invention, the solid phase support is selected from glass surfaces, plastic surfaces, silicon surfaces, solid organic polymers, cellulose/cellulose-based membranes, colloidal metal particles, or magnetic particles. Preferably, the solid phase support is a plastic surface. Most preferably, the solid phase support is a microtiter or ELISA plate. Microtiter or ELISA plates are conventional products, which are well known to the person skilled in the art.

The method of the invention follows a standard ELISA protocol. The general steps thereof are known to person skill in the art. Specifically, the cell-based ELISA protocol of the present invention can be performed comprising to the following exemplified steps:
- Breast cancer cells with a concentration of around 50 000 cells/well are cultured on ELISA plate overnight.
- The cells coated on the surface of the ELISA plate are rinsed twice with e.g., 100µl of a suitable buffer, such as Dulbecco's phosphate buffered saline (DPBS) with a suitable pH, e.g., pH7.4.
- To fix the cells on the surface of the ELISA plate, e.g., 100µl of a suitable fixing agent, such as 4% paraformaldehyde solution (PFA) are added thereafter to the cells for about 15 min at RT.
- Then, the fixation agent (e.g., PFA) is removed and the cells are washed twice with a suitable buffer, such as DPBS.
- Next, the cells on the surface of the ELISA plate are incubated with e.g., 20ug/ml PF4 for 1 h in RT to coat cells with PF4.
- Thereafter, the PF4 is removed, and the cells are washed three times with a suitable buffer, such as DPBS.

The ELISA plate is now coated with cancer cells, to which PFA has been adhered. The so prepared ELISA plate is now ready for analysis of biological samples.

Typical further steps of the method of the invention include:
- Addition of KKO or RTO or human sera of different concentrations diluted in a suitable buffer such as DPBS, with e.g. 7.5% goat serum for e.g. 1 h at RT;
- Removal of the antibodies and washing three times with a suitable buffer, such as DPBS;
- Addition of e.g. 100µl goat anti-mouse HRP antibody diluted 1 :50000 in a suitable buffer, such as DPBS and incubation with 7.5% goat serum for e.g. 1 h at RT;
- Removal of the goat anti-mouse antibodies and washing three times with a suitable buffer, such as DPBS;
- Addition of e.g. 100µl of a substrate for the enzyme label of the secondary antibody, such as 3,3',5,5'-Tetramethylbenzidine (TMB) solution, incubation for about 5 min in the dark to start the;
- Addition of e.g. in 100µl 1M H₂SO₄ to stop reaction;
- Measuring the optical density on the ELISA plate at 450nm absorbance wavelength.

As described hereinbefore, instead of whole cancer cells, there can also membranes of cancer cells be used in the method of the invention. Methods for obtaining cell membranes from a cell line are known to the person skilled in the art. E.g., after cell disruption, density gradient centrifugation may be utilized, thereby centrifuging cells with a fraction buffer in different speed and time, wherein the membrane of cells are isolated from other cell organelles for further purpose and use.

The biological sample used in the method of the invention is preferably selected from blood, serum, plasma, urine, or cerebrospinal fluid. More preferably, the biological sample is serum. Even more preferably, the biological sample is plasma. In a most preferred embodiment, the method of the invention is performed with a biological sample that has already been obtained from a subject, preferably a human.

Secondary antibodies suitable for use in the method of the invention are generally known to the person skilled in the art. Preferably, the secondary antibody used in the method of the invention is an anti-immunoglobulin (IgG) antibody that is optionally labeled with a detectable moiety. More preferably, the secondary antibody is selected from anti-mouse IgG antibodies for the detection of HIT-like RTO and KKO, and from anti-human IgG antibodies for the detection of human HIT antibodies.

For diagnostic applications, the detection antibody will typically be labelled with a detectable moiety. Numerous labels are available which can be generally grouped into the following categories:
(a) Radioisotopes, such as 35_{S}, 14_{C}, ¹²⁵I, ³H, and ¹³¹I. The antibody can be labeled with the radioisotope using the techniques described in Current Protocols in Immunology, Volumes 1 and 2, Gütigen et al., Ed., Wiley-Interscience. New York, New York. Pubs., (1991) for example, and radioactivity can be measured using scintillation counting.
(b) Fluorescent labels such as rare earth chelates (europium chelates) or fluorescein and its derivatives, rhodamine, and its derivatives, dansyl, Lissamine, phycoerythrin, and Texas Red are available. The fluorescent labels can be conjugated to the antibody using the techniques disclosed in Current Protocols in Immunology, supra for example. Fluorescence can be quantified using a fluorimeter.
(c) Various enzyme-substrate labels are available. The enzyme generally catalyses a chemical alteration of the chromogenic substrate which can be measured using various techniques. For example, the enzyme may catalyze a color change in a substrate, which can be measured spectrophotometrically. Alternatively, the enzyme may alter the fluorescence or chemiluminescence of the substrate. Techniques for quantifying a change in fluorescence are described above. The chemiluminescent substrate becomes electronically excited by a chemical reaction and may then emit light which can be measured (using a chemiluminometer, for example) or donates energy to a fluorescent acceptor. Examples of enzymatic labels include luciferases (e.g, firefly luciferase and bacterial luciferase; U.S. Patent No, 4,737,456), luciferin, 2,3-dihydrophthalazinediones, malate dehydrogenase, urease, peroxidase such as horseradish peroxidase (HRPO), alkaline phosphatase. 0-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like. Techniques for conjugating enzymes to antibodies are described in O'Sullivan et al., Methods for the Preparation of Enzyme-Antibody Conjugates for use in Enzyme Immunoassay, in Methods in Enzym. (ed Langone & H. Van Vunakis), Academic Press, New York, 73: 147-166 (1981).

Examples of enzyme-substrate combinations include, for example:
(i) Horseradish peroxidase (HRP) with hydrogen peroxidase as a substrate, wherein the hydrogen peroxidase oxidizes a dye precursor (e.g. orthophenylene diamine (OPD) or 3,3',5,5'-tetramethyl benzidine hydrochloride (TMB));
(ii) alkaline phosphatase (AP) with para-Nitrophenyl phosphate as chromogenic substrate; and
(iii) β-D-galactosidase (β-D-Gal) with a chromogenic substrate (e.g. β-nitrophenyl-β-D-galactosidase) or the fluorogenic substrate 4-methylumbellifery1-β-D-galactosidase.

Numerous other enzyme-substrate combinations are available to those skilled in the art. Most preferably, said anti-mouse IgG antibody used in the method of the invention is goat anti-mouse IgG Alexa 488, in particular for CLSM and flow-cytometry; or goat anti-mouse IgG HRP, in particular for ELISA, both for the detection of HIT-like RTO and KKO.

Even most preferably, said anti-human IgG antibody used in the method of the invention is goat anti-human IgG Alexa 488, in particular for CLSM and flow-cytometry; or goat anti-human IgG HRP, in particular for ELISA, both for the detection of human HIT Abs.

Methods for the detection of said labeled secondary anti-human antibodies are generally known in the art. Preferably, the detection of said labeled secondary anti-human antibody is performed with a method comprising optical density measurement, immunofluorescent detection using confocal laser scanning microscopy (CLMS), and flow cytometry.

More preferably, the detection is carried out by an assay such as an enzyme-linked immunosorbent assay (ELISA), a radioimmunoassay (RIA), an immunoradiometric assay (IRMA), a fluorescent immunoassay (FIA), a chemiluminescent immunoassay (CLIA), an electro-chemiluminescent immunoassay (ECL) or an agglutination assay.

Most preferably, the detection of the labeled secondary anti-human antibody is carried out by an ELISA assay.

The secondary antibody used in the method of the invention may be also an antibody that is not labeled. In such a case, the detection of the secondary antibody is performed by a method selected from the group comprising single-molecule force spectroscopy (SMFS)-based atomic force microscopy (AFM), Quartz crystal microbalance (QCM), *surface plasmon resonance (SPR),* Electrochemical impedance spectroscopy (EIS) and Dynamic light scattering (DLS).

In a further aspect, the invention relates to the use of the method according to any one of the preceding claims for diagnosing heparin-induced thrombocytopenia in a subject and to a method for diagnosing heparin-induced thrombocytopenia in a subject. Said use or method comprises the detection of heparin-induced thrombocytopenia antibodies (HIT Abs) in a biological sample according to the method described herein, wherein said biological sample has optionally been previously obtained from a subject and wherein the presence of HIT Abs in the biological sample is a positive indicator that said subject is suffering from heparin-induced thrombocytopenia.

If the detection of the secondary IgG antibody, i.e. the presence of HIT Abs in the biological sample in the method of the invention is measured with an ELISA assay, the optical density (OD) is measured on the microtiter plates. According to current standards in clinical laboratories, if the measured OD is < 0.5, the tested subject is not suspected to suffer from heparin-induced thrombocytopenia. If the measured OD is ≥ 0.5, the tested subject is suspected to suffer from heparin-induced thrombocytopenia.

In a preferred embodiment of the use or method for diagnosing heparin-induced thrombocytopenia, said use or method further comprises the step of comparing the detected amount of HIT Abs in the biological sample from a subject with an amount of HIT Abs characteristic of a normal control subject; whereby an elevated amount of HIT Abs in said biological sample from the subject relative to the normal control is a positive indicator that said subject is suffering from heparin-induced thrombocytopenia. As described above, the detection of the labeled secondary IgG antibody, i.e. of HIT Abs, can be performed with a method comprising an optical density measurement, immunofluorescent detection using confocal laser scanning microscopy (CLMS) and flow cytometry in said biological sample from a subject and a sample from the normal control subject.

Provided herein but not part of the invention is a kit comprising
- a solid phase support comprising cancer cells on its surface,
- a washing solution to remove non-HIT Abs from the solid phase support after contacting the solid phase support with a biological sample,
- appropriate reaction buffers for performing an assay selected from the group consisting of: an enzyme linked immunosorbent assay (ELISA), a radioimmunoassay (RIA), an immuno radiometric assay (IRMA), a fluorescent immunoassay (FIA), a chemiluminescent immunoassay (CLIA), an electro-chemiluminescent immunoassay (ECL) and an agglutination assay,
- a manual comprising instructions for using the kit, and
- optionally a positive control and/or a negative control.

Said kit may comprise further ingredients such as platelet factor 4 (PF4) in an appropriate solution and concentration and a secondary anti-human antibody, which is preferably labeled and is capable of binding to the bound HIT Abs on the cells or cell membranes on the solid phase support.

As demonstrated in the working examples, the invention has multiple advantages that overcome the limitations of conventional methods available in the prior art. The method of the invention uses in a most preferred embodiment the ELISA-based detection of HIT Abs bound to cancer cells or cancer cell membranes on the surface of a solid phase support. The ELISA-Method of the invention
- shows high sensitivity and specificity,
- is relatively rapid,
- is easy to perform with less complications,
- shows a good cost-effectiveness,
- does not require fresh human blood, but simply uses a robust cell line,
- enables easy detection by using different techniques,
- enables the testing of large batches of samples in a single run,
- shows an excellent prediction,
- allows IgG-only detection,
- requires only one type of labeled-secondary antibody, and
- is very reliable in the conclusion, if patients have HIT, because the method allows a clear distinction between HIT and non-HIT signal.

### Examples of the invention

### 1. ELISA assay

**Fig. 1 (C)** and **(D)** illustrate that the invention as an add-in of the traditional ELISA for detection of HIT Abs. Instead of coating PF4 or PF4/Heparin antigens directly on the ELISA plate, breast cancer cells were first cultured on the ELISA plate. Subsequently, PF4 or PF4/Heparin antigens for binding HIT antibodies (HIT Abs) on said cells are coated on the cancer cells or their cell membranes. The next steps of bindings of HIT antibodies to cells and the adhesion of labeled antibodies were performed following the standard ELISA protocol. The binding of PF4 or PF4/Heparin antigens to the cancer cells or their cell membranes changes the conformation and net of surface changes of said antigens and induces the binding of epitopes of HIT Abs but not of non-HIT Abs (Fig. 1C). As a result, a clear distinction signal between HIT and non-HIT antibodies was obtained (Fig. 1D). The method comprises i) culturing breast cancer cells on ELISA plates, ii) binding of antigens (PF4 or PF4/H complexes) to said breast cancer cells, iii) binding of HIT Abs in diluted sera to PF4-bound cells, iv) adhering a labeled secondary anti-human antibody that is capable of binding to the bound HIT Abs on breast cancer cells or their membranes, and iv) detecting the presence of the labeled secondary anti-human antibody. Thereby, the HIT Abs bound to the labeled secondary antibody were identified. The results shown in Fig. 1D were obtained with breast cancer cell line MDA MB 231.

For comparison, Fig. 1 (A) and (B) is the schematic illustration of a traditional ELISA for detection of HIT Abs. PF4/Heparin complexes are coated directly on a microtiter plate before incubating the microtiter plate with human sera containing anti-PF4/Heparin antibodies (Fig. 1A). The surface of the microtiter plate is thereby coated with anti-PF4/Heparin antibodies. Thereafter, to these anti-PF4/Heparin antibodies coated on the surface of the microtiter plates, a labeled anti-human IgG is bound, which subsequently causes a color change on the substrate, which is detected by a photometer and expressed as optical density (OD). However, this method allows binding of both HIT and non-HIT antibodies as around 50% detected OD signal is overlapped (Fig. 1B).

### Results

While traditional ELISA induces about 50% overlap of the detected OD signal of HIT Abs and non-HIT Abs, the present invention allows a clear distinction and an extremely effective differentiation between HIT Abs and non-HIT Abs (nearly 100%). Only one labeled secondary antibody is required for the detection. Breast cancer cells (e.g. cell line: MDA MB 231) are easy to culture and multiple passages can be used. The signal can be easily detected by different techniques such as ELISA reader, CLMS, and FACS, etc. using almost the same protocol. The ELISA cell (membrane) assay of the invention provides a high specificity, a short protocol, is easy to handle and lowers the cost for a more reliable detection of HIT Abs. Multiple limitations from other methods are eliminated by the invention, including high costs, low accuracy, the requirement of fresh human blood/platelets, complications, toxicity and specialized physicians.

### 2. Visualization of binding of HIT antibodies on cells

**Fig. 2** shows a visualization of the binding of HIT antibodies bound on breast cancer cells by immunofluorescent detection using confocal laser scanning microscopy (CLMS). Cells were cultured on ELISA plates and coated with 20 µg/ml PF4 or PF4/Heparin antigens (Fig. 2A). Binding of RTO and KKO on the cells was detected *via* binding of secondary anti-mouse antibody-Alexa488. To visualize cell nuclei, cells were stained with DAPI blue (Fig. 2B) and imaged by CLMS.

No fluorescent signal was detected on cells alone, cells coated with labeled anti-mouse antibody-Alexa488, and PF4/H complexes coated cells in the presence of the labeled antibody (Fig. 2B). Interestingly, on cells coated with PF4 or PF4/H antigens, RTO showed minimal binding while KKO bind strongly as shown by a high fluorescent signal in bright on cells (Fig. 2B). Quantification of fluorescent area (green signal) showed a clear distinction between KKO and RTO (Fig. 2C).

### 4. Quantification of binding of HIT antibodies on cells by flow cytometry

**Fig 3** shows the detection of HIT Abs bound on breast cancer cells by flow cytometry. In the above experiments, cells were cultured on an ELISA plate in 2-dimension. Now, the fluorescent signal difference between KKO and RTO was further quantified, when the cells were adhesion-free in the medium. The cells were incubated with PF4 and then the suspension was removed after centrifugation. Then, RTO or KKO was added to the cell for binding. Next, the suspension was again removed after centrifugation. Finally, the anti-mouse-antibody-Alexa488 sample was incubated with cells. After removing the suspension buffer, cells were suspended in the medium for flow cytometry measurements. Only with the PF4 pre-incubation, both RTO and KKO can bind to cancer cells. In the same concentration of RTO and KKO with PF4 incubation, the fluorescent signal from KKO was stronger than RTO and shifted far away from the labeled antibody controls. RTO, on the other hand, showed much weaker fluorescent signal compared to KKO and even partially overlapped with the control cell only. The results show a clear and strong difference in fluorescent signal between KKO and RTO (Fig. 3). This indicates that the developed cancer cell assay also allows to detect HIT abs by flow cytometry.

### 4. Detection of HIT antibodies by single-molecule force spectroscopy

**Fig. 4** shows the different binding between HIT and non-HIT antibodies on bound-PF4 antigen on breast cancer cells obtained by single-molecule force spectroscopy (SMFS)-based atomic force microscopy (AFM). As a proof of principle, two types of commercially available well-characterized monoclonal HIT-like murine aPF4/H Abs including RTO and KKO were tested. The RTO has binding characteristics similar to the non-HIT Abs while the KKO mimics the HIT Abs.²⁷⁻²⁹ Although both RTO and KKO bind to PF4/H complexes via Fab parts, KKO antibodies contain a Fc parts that bridge/activate platelets *via* FcyRlla receptors on platelets, resulting in HIT, whereas RTO does not contain platelet activating receptor.

The single RTO or KKO was immobilized on the tip for interacting with PF4 or PF4/H antigens coated on breast cancer cells (Fig. 4A). Upon moving up and down, the adhesion force allows measuring the interaction forces as well as the frequency of interactions. Analysis of the frequency of interactions when cells were coated with different PF4 concentrations up to 50 µg/ml or with preformed PF4/Heparin complexes shows a much higher frequency of interaction (count number) for KKO than RTO (Fig. 4B). The results indicate that the HIT-antibody KKO can be distinguished from the non-HIT antibody RTO with a labelled-free technique.

### References

1 Rice, L. Heparin-induced thrombocytopenia: myths and misconceptions (that will cause trouble for you and your patient). Arch Intern Med 164, 1961-1964, doi:10.1001/archinte.164.18.1961 (2004).
2 Nguyen, T. H. et al. Characterization of the interaction between platelet factor 4 and homogeneous synthetic low molecular weight heparins. J Thromb Haemost 18, 390-398, doi:10.1111/jth. 14657 (2020).
3 Warkentin T. E. , C. B. H., Greinacher A. Heparin-induced Thrombocytopenia: To w a rds Consensus. Thrombosis and haemostasis 79, 1-7 (1998).
4 Warkentin, T. E., Basciano, P. A., Knopman, J. & Bernstein, R. A. Spontaneous heparin-induced thrombocytopenia syndrome: 2 new cases and a proposal for defining this disorder. Blood 123, 3651-3654, doi:10.1182/blood-2014-01-549741 (2014).
5 Cummins, D., Halil, O. & Amin, S. Which patients undergoing cardiopulmonary bypass should be assessed for development of heparin-induced thrombocytopenia? Thromb Haemost 73, 890 (1995).
6 Go, A. S. et al. Heart disease and stroke statistics--2013 update: a report from the American Heart Association. Circulation 127, e6-e245, doi:10.1161/CIR.0b013e31828124ad (2013).
7 Thachil J, T. N., Gando S, Falanga A, Cattaneo M, Levi M, et al.. ISTH interim guidance on recognition and managment of coagulopathy in COVID-19. J Thromb Haemost. , doi:doi: 10.1111/JTH.14810. (2020).
8 Cattaneo, M. et al. Pulmonary Embolism or Pulmonary Thrombosis in COVID-19? Is the Recommendation to Use High-Dose Heparin for Thromboprophylaxis Justified? Thromb Haemost, doi:10.1055/s-0040-1712097 (2020).
9 Tang, N. et al. Anticoagulant treatment is associated with decreased mortality in severe coronavirus disease 2019 patients with coagulopathy. Journal of thrombosis and haemostasis : JTH 18, 1094-1099, doi:10.1111/jth.14817 (2020).
10 Yin, S., Huang, M., Li, D. & Tang, N. Difference of coagulation features between severe pneumonia induced by SARS-CoV2 and non-SARS-CoV2. J Thromb Thrombolysis, doi:10.1007/s11239-020-02105-8 (2020).
11 Dragonetti, D., Guarini, G. & Pizzuti, M. Detection of anti-heparin-PF4 complex antibodies in COVID-19 patients on heparin therapy. Blood Transfus 18, 328, doi:10.2450/2020.0164-20 (2020).
12 Warkentin, T. E. et al. Heparin-induced thrombocytopenia in patients treated with low-molecular-weight heparin or unfractionated heparin. N Engl J Med 332, 1330-1335, doi:10.1056/NEJM199505183322003 (1995).
13 Karina Althaus, I. M., Jan Zlamal, Lisann Pelzl, Anurag Singh, Helene Häberle, Martin Mehrländer, Stefanie Hammer, Harald Schulze, Michael Bitzer, Nisar Malek, Dominik Rath, Hans Bösmüller, Bernard Nieswandt, Meinrad Gawaz, Tamam Bakchoul, Peter Rosenberger. Antibody-induced procoagulant platelets in severe COVID-19 infection. Blood, doi:https://doi.org/10.1182/blood.2020008762 (2021).
14 Michigan, M. M.-U. o. New cause of COVID-19 blood clots identified. Science News, doi:https://www.sciencedaily.com/releases/2020/11/201102142254.htm (November 2, 2020).
15 Ning Tang, D. L., Xiong Wang, Ziyong Sun. Abnormal coagulation parameters are associated with poor prognosis in patients with novel coronavirus pneumonia. J. Thromb Heamost. (2020).
16 Xiaobo Yang, Q. Y., Yaxin Wang, Yongran Wu, Jiqian Xu, Yuan Yu, and You Shang. Thrombocytopenia and Its Association with Mortality in Patients with COVID-19. J Thromb Haemost. (2020).
17 Cui, S., Chen, S., Li, X., Liu, S. & Wang, F. Prevalence of venous thromboembolism in patients with severe novel coronavirus pneumonia. Journal of thrombosis and haemostasis : JTH, doi:10.1111/jth. 14830 (2020).
18 Warkentin, T. E., Arnold, D. M., Nazi, I. & Kelton, J. G. The platelet serotonin-release assay. Am J Hematol 90, 564-572, doi:10.1002/ajh.24006 (2015).
19 Warkentin, T. E. et al. Laboratory testing for heparin-induced thrombocytopenia: a conceptual framework and implications for diagnosis. J Thromb Haemost 9, 2498-2500, doi:10.1111/j.1538-7836.2011.04536.x (2011).
20 Greinacher, A. et al. Laboratory Diagnosis of Heparin-Associated Thrombocytopenia and Comparison of Platelet-Aggregation Test, Heparin-Induced Platelet Activation Test, and Platelet Factor-4 Heparin Enzyme-Linked-Immunosorbent-Assay. Transfusion 34, 381-385, doi:DOI 10.1046/j.1537-2995.1994.34594249047.x (1994).
21 Nagler, M., Bachmann, L. M., ten Cate, H. & ten Cate-Hoek, A. Diagnostic value of immunoassays for heparin-induced thrombocytopenia: a systematic review and meta-analysis. Blood 127, 546-557, doi:10.1182/blood-2015-07-661215 (2016).
22 Nguyen, T. H. et al. Reactivity of platelet-activating and nonplatelet-activating anti-PF4/heparin antibodies in enzyme immunosorbent assays under different conditions. J Thromb Haemost 17, 1113-1119, doi:10.1111/jth.14455 (2019).
23 Warkentin, T. E. & Heddle, N. M. Laboratory diagnosis of immune heparin-induced thrombocytopenia. Curr Hematol Rep 2, 148-157 (2003).
24 Meyer, O., Salama, A., Pittet, N. & Schwind, P. Rapid detection of heparin-induced platelet antibodies with particle gel immunoassay (ID-HPF4). Lancet 354, 1525-1526, doi:10.1016/S0140-6736(99)03625-9 (1999).
25 Sachs, U. J., von Hesberg, J., Santoso, S., Bein, G. & Bakchoul, T. Evaluation of a new nanoparticle-based lateral-flow immunoassay for the exclusion of heparin-induced thrombocytopenia (HIT). Thromb Haemost 106, 1197-1202, doi:10.1160/TH-11-06-0390 (2011).
26 Favaloro, E. J. Laboratory tests for identification or exclusion of heparin induced thrombocytopenia: HIT or miss? Am J Hematol 93, 308-314, doi:10.1002/ajh.24979 (2018).
27 Rauova, L. et al. Role of platelet surface PF4 antigenic complexes in heparin-induced thrombocytopenia pathogenesis: diagnostic and therapeutic implications. Blood 107, 2346-2353, doi:10.1182/blood-2005-08-3122 (2006).
28 Jourdy, Y. et al. Prospective evaluation of automatized PF4/heparin immunoassays HemosIL HIT-ab (PF4-H) for the diagnosis of heparin-induced thrombocytopenia. Int J Lab Hematol 37, 244-252, doi:10.1111/ijlh.12275 (2015).
29 Sachais, B. S. et al. Dynamic antibody-binding properties in the pathogenesis of HIT. Blood 120, 1137-1142, doi:10.1182/blood-2012-01-407262 (2012).
30 Vayne, C. et al. Characterization of New Monoclonal PF4-Specific Antibodies as Useful Tools for Studies on Typical and Autoimmune Heparin-induced Thrombocytopenia. Thromb Haemost, doi:10.1055/s-0040-1717078 (2020).
31 Arepally, G. M. et al. Characterization of a murine monoclonal antibody that mimics heparin-induced thrombocytopenia antibodies. Blood (2000) 95 (5): 1533-1540, doi.org/10.1182/blood.V95.5.1533.005k01_1533_1540.
32 Li, Z.; Ruan, J.; Zhuang, X., Effective capture of circulating tumor cells from an S180-bearing mouse model using electrically charged magnetic nanoparticles. J Nanobiotechnology
33 Mulhall HJ, Hughes MP, Kazmi B, Lewis MP, Labeed FH. Epithelial cancer cells exhibit different electrical properties when cultured in 2D and 3D, environments. Biochim Biophys Acta. 2013;1830:5136-41.

## Claims

1. An in vitro method for the detection of heparin-induced thrombocytopenia antibodies (HIT Abs) in a biological sample comprising
i) providing a solid phase support, wherein said solid support is coated with cells or cell membranes on the surface,
ii) binding of platelet factor 4 (PF4) to said cells or cell membranes on the solid phase support,
iii) incubating the solid phase support of step ii) with a biological sample,
iv) binding of HIT Abs which are present in said biological sample to PF4 on said cells or cell membranes on the solid phase support,
v) adhering a secondary anti-human antibody, which is preferably labeled and is capable of binding to the bound HIT Abs on said cells on the solid phase support, and
vi) detecting the presence of the secondary anti-human antibody,
wherein the detection of said secondary anti-human antibody is a positive indicator for the presence of HIT Abs in the biological sample, **characterized in that** said cells or cell membranes on the solid phase support, are cancer cells or membranes from cancer cells.

2. The method of claim 1, wherein said solid phase support is selected from glass surfaces, plastic surfaces, silicon surfaces, solid organic polymers, cellulose/cellulose-based membranes, colloidal metal particles or magnetic particles.

3. The method of claim 1 or 2, wherein said solid phase support is a microtiter plate.

4. The method according to any one of the preceding claims, wherein said biological sample is selected from a blood sample, a serum sample, a plasma sample, a cerebrospinal fluid sample or a urine sample.

5. The method according to any one of the preceding claims, wherein said biological sample is human serum.

6. The method according to any one of the preceding claims, wherein said cancer cells or cancer cell membranes are, e.g. obtained from cancer cells selected from malignant (and preferably solid) tumors of epithelial or mesenchymal cells, breast cancer, prostate cancer, pancreatic cancer, adrenal cancer, melanoma, lung cancer, colon cancer, leukemia (a liquid or non-solid tumor), soft tissue and bone sarcomas, neuroendocrine tumors such as islet cell carcinoma or medullary carcinoma of the thyroid, squamous carcinomas (particularly of the head and neck), adenocarcinomas and gliosarcomas such as glioblastoma multiforme.

7. The method according to any one of the preceding claims, wherein said cancer cells are cells from the breast cancer cell line MDA-MB 231.

8. The method according to any one of the preceding claims, wherein said secondary antibody is selected from anti-mouse IgG antibodies, such as goat anti-mouse IgG alexa 488 or goat anti-mouse IgG HRP; and from anti-human IgG antibodies, such as such as goat anti-human IgG alexa 488 or goat anti-human IgG HRP.

9. The method according to any one of the preceding claims, wherein said secondary antibody is labeled with a label selected from
(a) Radioisotopes, such as 35_{S}, 14_{C}, ¹²⁵I, ³H, and ¹³¹I;
(b) Fluorescent labels such as rare earth chelates (europium chelates) or fluorescein and its derivatives, rhodamine and its derivatives, dansyl, Lissamine, phycoerythrin and Texas Red; and
(c) enzymatic labels including luciferases, luciferin, 2,3-dihydrophthalazinediones, malate dehydrogenase, urease, peroxidase such as horseradish peroxidase (HRPO), alkaline phosphatase, O-galactosidase, glucoamylase, lysozyme, saccharide oxidases (e.g., glucose oxidase, galactose oxidase, and glucose-6-phosphate dehydrogenase), heterocyclic oxidases (such as uricase and xanthine oxidase), lactoperoxidase, microperoxidase, and the like.

10. The method according to any one of the preceding claims, wherein the detection of said labeled secondary anti-human antibody is performed with a method comprising optical density measurement, immunofluorescent detection using confocal laser scanning microscopy (CLMS) and flow cytometry.

11. The method of claims 1 to 8, wherein the secondary antibody is not labeled and wherein detection of the secondary antibody is performed by a method selected from group comprising single-molecule force spectroscopy (SMFS)-based atomic force microscopy (AFM), quartz crystal microbalance (QCM), surface plasmon resonance (SPR), electrochemical impedance spectroscopy (EIS) and dynamic light scattering (DLS).

12. The method according to any one of the preceding claims, wherein the detecting is carried out by an assay such as an enzyme linked immunosorbent assay (ELISA), a radioimmunoassay (RIA), an immuno radiometric assay (IRMA), a fluorescent immunoassay (FIA), a chemiluminescent immunoassay (CLIA), an electro-chemiluminescent immunoassay (ECL) or an agglutination assay.

13. Use of the method according to any one of the preceding claims for diagnosing heparin-induced thrombocytopenia in a subject.

14. A method for diagnosing heparin-induced thrombocytopenia in a subject, wherein said method comprises the detection of heparin-induced thrombocytopenia antibodies (HIT Abs) in a biological sample according to any one of claims 1 to 11 which has been previously been obtained from a subject, wherein the presence of HIT Abs in the biological sample is a positive indicator that said subject is suffering from heparin-induced thrombocytopenia, wherein said method of diagnosing optionally further comprises the step of comparing the detected amount of HIT Abs in the biological sample from a subject with an amount of HIT Abs characteristic of a normal control subject; whereby an elevated amount of HIT Abs in said biological sample from the subject relative to the normal control is a positive indicator that said subject is suffering from heparin-induced thrombocytopenia.

## Patentansprüche

1. In-vitro-Verfahren zum Nachweis von Heparininduzierte-Thrombozytopenie-Antikörpern (HIT-Abs) in einer biologischen Probe, umfassend
i) Bereitstellen eines Festphasenträgers, wobei der Feststoffträger auf der Oberfläche mit Zellen oder Zellmembranen beschichtet ist,
ii) Binden von PF4 (Platelet Factor 4) an die Zellen oder Zellmembranen auf dem Festphasenträger,
iii) Inkubieren des Festphasenträgers aus Schritt ii) mit einer biologischen Probe,
iv) Binden von HIT-Abs, die in der biologischen Probe vorhanden sind, an PF4 auf den Zellen oder Zellmembranen auf dem Festphasenträger,
v) Anhaften eines sekundären Anti-Mensch-Antikörpers, der vorzugsweise markiert ist und an die gebundenen HIT-Abs auf den Zellen auf dem Festphasenträger binden kann, und
vi) Nachweisen des Vorliegens des sekundären Anti-Mensch-Antikörpers,
wobei der Nachweis des sekundären Anti-Mensch-Antikörpers einen positiven Indikator für das Vorhandensein von HIT-Abs in der biologischen Probe darstellt, **dadurch gekennzeichnet, dass** es sich bei den Zellen oder Zellmembranen auf dem Festphasenträger um Krebszellen bzw. Membranen von Krebszellen handelt.

2. Verfahren nach Anspruch 1, wobei der Festphasenträger aus Glasflächen, Kunststoffflächen, Siliciumdioxidflächen, organischen Feststoffpolymeren, Cellulosemembranen bzw. Membranen auf Cellulosebasis, Metallkolloiden oder magnetischen Partikeln ausgewählt sind.

3. Verfahren nach Anspruch 1 oder 2, wobei es sich bei dem Festphasenträger um eine Mikrotiterplatte handelt.

4. Verfahren gemäß einem der vorhergehenden Ansprüche, wobei die biologische Probe aus einer Blutprobe, einer Serumprobe, einer Plasmaprobe, einer Liquorprobe oder einer Urinprobe ausgewählt ist.

5. Verfahren gemäß einem der vorhergehenden Ansprüche, wobei es sich bei der biologischen Probe um Humanserum handelt.

6. Verfahren gemäß einem der vorhergehenden Ansprüche, wobei die Krebszellen oder Krebszellmembranen z. B. von Krebszellen erhalten werden, die aus bösartigen (und bevorzugt soliden) Tumoren von Epithel- oder Mesenchymzellen, Brustkrebs, Prostatakrebs, Bauchspeicheldrüsenkrebs, Nebennierenkrebs, Melanom, Lungenkrebs, Darmkrebs, Leukämie (ein flüssiger oder nichtsolider Tumor), Weichteil- und Knochensarkomen, neuroendokrinen Tumoren wie Inselzellkarzinom oder medullärem Schilddrüsenkarzinom, Plattenepithelkarzinomen (besonders im Kopf- und Halsbereich), Adenokarzinomen und Gliosarkomen wie Glioblastoma multiforme ausgewählt sind.

7. Verfahren gemäß einem der vorhergehenden Ansprüche, wobei es sich bei den Krebszellen um Zellen aus der Brustkrebszelllinie MDA-MB 231 handelt.

8. Verfahren gemäß einem der vorhergehenden Ansprüche, wobei der sekundäre Antikörper aus Anti-Maus-IgG-Antikörpern wie Ziege-Anti-Maus-IgG-Alexa 488 oder Ziege-Anti-Maus-IgG-HRP; sowie aus Anti-Mensch-IgG-Antikörpern wie Ziege-Anti-Mensch-IgG-Alexa 488 oder Ziege-Anti-Mensch-IgG-HRP ausgewählt ist.

9. Verfahren gemäß einem der vorhergehenden Ansprüche, wobei der sekundäre Antikörper mit einer Markierung, die aus
(a) Radioisotopen wie 35_{S}, 14_{C}, ¹²⁵I, ³H und ¹³¹I;
(b) Fluoreszenzmarkierungen wie Seltene-Erden-Chelaten (Europiumchelate) oder Fluorescein und seinen Derivaten, Rhodamin und seinen Derivaten, Dansyl, Lissamin, Phycoerythrin und Texas Red; und
(c) enzymatischen Markierungen, einschließlich Luciferasen, Luciferin, 2,3-Dihydrophthalazindionen, Malatdehydrogenase, Urease, Peroxidase wie Meerrettich-Peroxidase (HRPO), alkalischer Phosphatase, O-Galactosidase, Glucoamylase, Lysozym, Saccharidoxidasen (z. B. Glucose-Oxidase, Galactose-Oxidase und Glucose-6-phosphat-Dehydrogenase), heterocyclischer Oxidasen (wie Uricase und Xanthin-Oxidase), Lactoperoxidase, Mikroperoxidase und dergleichen, ausgewählt sind, markiert ist.

10. Verfahren gemäß einem der vorhergehenden Ansprüche, wobei der Nachweis des markierten sekundären Anti-Mensch-Antikörpers mit einem Verfahren erfolgt, das Messung optischer Dichte, Immunfluoreszenznachweis mit CLMS (Confocal Laser Scanning Microscopy) und Durchflusszytometrie umfasst.

11. Verfahren nach Anspruch 1 bis 8, wobei der sekundäre Antikörper nicht markiert ist und wobei der sekundäre Antikörper mit einem Verfahren nachgewiesen wird, das aus einer Gruppe, die auf SMFS (Single-Molecule Force Spectroscopy) beruhende Rasterkraftmikroskopie (Atomic Force Microscopy, AFM), QCM (Quartz Crystal Microbalance), Oberflächenplasmonenresonanz (Surface Plasmon Resonance, SPR), Elektrochemische-Impedanz-Spektroskopie (EIS) und dynamische Lichtstreuung (DLS) umfasst, ausgewählt ist.

12. Verfahren gemäß einem der vorhergehenden Ansprüche, wobei das Nachweisen mit einem Assay wie einem ELISA (Enzyme Linked Immunosorbent Assay), einem RIA (Radioimmunoassay), einem IRMA (Immuno Radiometrie Assay), einem FIA (Fluorescent Immunoassay), einem CLIA (Chemiluminescent Immunoassay), einem ECL (Electro-Chemiluminescent Immunoassay) oder einem Agglutinierung-Assay durchgeführt wird.

13. Verwendung des Verfahren gemäß einem der vorhergehenden Ansprüche zur Diagnose von heparininduzierter Thrombozytopenie bei einem Individuum.

14. Verfahren zur Diagnose von heparininduzierter Thrombozytopenie bei einem Individuum, wobei das Verfahren den Nachweis von Heparininduzierte-Thrombozytopenie-Antikörpern (HIT-Abs) in einer biologischen Probe gemäß einem der Ansprüche 1 bis 11, die zuvor von einem Individuum erhalten wurde, wobei das Vorhandensein von HIT-Abs in der biologischen Probe ein positiver Indikator dafür ist, dass das Individuum an heparininduzierter Thrombozytopenie leidet, wobei das Diagnoseverfahren gegebenenfalls ferner den Schritt umfasst, bei dem die nachgewiesene Menge an HIT-Abs in der biologischen Probe mit einer HIT-Abs-Menge verglichen wird, die für eine Normalindividuumkontrolle charakteristisch ist; womit eine erhöhte HIT-Abs-Menge in der biologischen Probe aus dem Individuum relativ zur Normalkontrolle einen positiven Indikator dafür darstellt, dass das Individuum an heparininduzierter Thrombozytopenie leidet.

## Revendications

1. Procédé *in vitro* pour la détection des anticorps de la thrombocytopénie induite par l'héparine (HIT Abs) dans un échantillon biologique comprenant
i) fourniture d'un support en phase solide, dans lequel ledit support solide est recouvert par des cellules ou des membranes cellulaires sur la surface,
ii) liaison du facteur plaquettaire 4 (PF4) auxdites cellules ou membranes cellulaires sur le support de phase solide,
iii) incubation du support en phase solide de l'étape ii) avec un échantillon biologique,
iv) liaison de HIT Abs présent dans cet échantillon biologique à PF4 sur lesdites cellules ou membranes cellulaires sur le support en phase solide,
v) adhérence d'un anticorps secondaire anti-humain, qui est de préférence marqué et capable de se lier à HIT Abs sur lesdites cellules sur le support de phase solide, et
vi) détection de la présence de l'anticorps secondaire anti-humain,
dans lequel la détection dudit anticorps secondaire anti-humain est un indicateur positif de la présence de HIT Abs dans l'échantillon biologique, **caractérisé en ce que** lesdites cellules ou membranes cellulaires sur le support de phase solide sont des cellules cancéreuses ou des membranes provenant de cellules cancéreuses.

2. Procédé selon la revendication 1, dans lequel ledit support de phase solide est sélectionné parmi les surfaces en verre, les surfaces en plastique, les surfaces en silicone, les polymères organiques solides, les membranes de cellulose/à base de cellulose, les particules métalliques colloïdales ou les particules magnétiques.

3. Procédé selon la revendication 1 ou 2, dans lequel ledit support en phase solide est une plaque de microtitration.

4. Procédé selon l'une quelconque des revendications précédentes, dans lequel ledit échantillon biologique est sélectionné parmi un échantillon de sang, un échantillon de sérum, un échantillon de plasma, un échantillon de fluide céphalorachidien ou un échantillon d'urine.

5. Procédé selon l'une quelconque des revendications précédentes, dans lequel ledit échantillon biologique est du sérum humain.

6. Procédé selon l'une quelconque des revendications précédentes, dans lequel lesdites cellules cancéreuses ou membranes de cellules cancéreuses sont, par exemple, obtenues à partir de cellules cancéreuses sélectionnées parmi des tumeurs malignes (et de préférence solides) de cellules épithéliales ou mésenchymateuses, un cancer du sein, un cancer de la prostate, un cancer du pancréas, un cancer de la glande surrénale, un mélanome, un cancer du poumon, un cancer du côlon, une leucémie (tumeur liquide ou non solide), des sarcomes des tissus mous et des os, des tumeurs neuroendocrines telles que le carcinome des cellules des îlots ou un carcinome médullaire de la thyroïde, des carcinomes épidermoïdes (en particulier de la tête et du cou), des adénocarcinomes et des gliosarcomes tels que le glioblastome multiforme.

7. Procédé selon l'une quelconque des revendications précédentes, dans lequel lesdites cellules cancéreuses sont des cellules de la lignée cellulaire de cancer du sein MDA-MB 231.

8. Procédé selon l'une quelconque des revendications précédentes, dans lequel ledit anticorps secondaire est sélectionné parmi des anticorps IgG anti-souris, tels que l'IgG anti-souris de chèvre alexa 488 ou l'IgG HRP anti-souris de chèvre ; et des anticorps IgG anti-humains, tels que l'IgG anti-humain de chèvre alexa 488 ou l'IgG HRP anti-humain de chèvre.

9. Procédé selon l'une quelconque des revendications précédentes, dans lequel ledit anticorps secondaire est marqué avec un marqueur choisi parmi
(a) des radio-isotopes, tels que 35_{S}, 14_{C}, ¹²⁵I, ³H et ¹³¹I ;
(b) des marqueurs fluorescents tels que les chélates de terres rares (chélates d'europium) ou la fluorescéine et ses dérivés, la rhodamine et ses dérivés, la dansyle, la lissamine, la phycoérythrine et le Texas Red ; et
(c) des marqueurs enzymatiques comprenant la luciférase, la luciférine, la 2,3-dihydrophtalazinedione, la malate déshydrogénase, l'uréase, une peroxydase telle que la peroxydase de raifort (HRO), la phosphatase alcaline, la O-galactosidase, la glucoamylase, le lysozyme, les saccharide oxydases (p. ex. glucose oxydase, galactose oxydase et glucose-6-phosphate déshydrogénase), les oxydases hétérocycliques (telles que l'uricase et la xanthine oxydase), la lactoperoxydase, la microperoxydase, et similaire.

10. Procédé selon l'une quelconque des revendications précédentes, dans lequel la détection dudit anticorps secondaire anti-humain marqué est effectuée avec un procédé comprenant la mesure de la densité optique, la détection par immunofluorescence en utilisant une microscopie à balayage laser confocal (CLMS) et la cytométrie de flux.

11. Procédé selon les revendications 1 à 8, dans lequel l'anticorps secondaire n'est pas marqué et dans lequel la détection de l'anticorps secondaire est effectuée par un procédé sélectionné dans un groupe comprenant la microscopie à force atomique (AFM) basée sur la spectroscopie de force d'une molécule unique (SMFS), la microbalance à cristal de quartz (QCM), la résonance de plasmon de surface (SPR), la spectroscopie d'impédance électrochimique (EIS) et la diffusion dynamique de lumière (DLS).

12. Procédé selon l'une quelconque des revendications précédentes, dans lequel la détection est effectuée par un dosage tel qu'un dosage immunoenzymatique (ELISA), un dosage radioimmunologique (RIA), un dosage immuno-radiométrique (IRMA), un immunodosage fluorescent (FIA), un immunodosage chimioluminescent (CLIA), un immunodosage par électrochimioluminescence (ECL) ou un dosage d'agglutination.

13. Utilisation du procédé selon l'une quelconque des revendications précédentes pour le diagnostic d'une thrombocytopénie induite par l'héparine chez un sujet.

14. Procédé de diagnostic de thrombocytopénie induite par l'héparine chez un sujet, dans lequel ledit procédé comprend la détection d'anticorps de thrombocytopénie induite par l'héparine (HIT Abs) dans un échantillon biologique selon l'une quelconque des revendications 1 à 11 qui a été précédemment obtenu auprès d'un sujet, dans lequel la présence de HIT Abs dans l'échantillon biologique est un indicateur positif que ledit sujet souffre de thrombocytopénie induite par l'héparine, dans lequel ledit procédé de diagnostic comprend éventuellement également l'étape de comparaison de la quantité détectée de HIT Abs dans l'échantillon biologique d'un sujet avec une quantité de HIT Abs caractéristique d'un sujet témoin normal ; moyennant quoi une quantité élevée de HIT Abs dans ledit échantillon biologique du sujet par rapport au témoin normal est un indicateur positif indiquant que ledit sujet souffre d'une thrombocytopénie induite par l'héparine.
